# NEW EUROPEAN PATENT SPECIFICATION

(11) **EP 0 862 419 B2**
(45) Date of publication and mention of the opposition decision: **17.11.2010**
(45) Mention of the grant of the patent: 03.05.2000
(21) Application number: 96938326.4
(22) Date of filing: 11.11.1996
(51) Int. Cl.: A61K 9/16, A61K 9/00, A61K 39/15, A61K 39/165, A61K 48/00

(54) **MICROENCAPSULATED DNA FOR VACCINATION AND GENE THERAPY**
MIKROVERKAPSELTE DNA ZUR IMPFUNG UND GENTHERAPIE
ADN MICROENCAPSULE S'APPLIQUANT DANS DES PROCEDES DE VACCINATION ET DE THERAPIE GENIQUE

(30) Priority: 09.11.1995 GB 9523019; 31.01.1996 GB 9601929
(43) Date of publication of application: 09.09.1998
(60) Divisional application: 99113415.6
(73) Proprietor: MICROBIOLOGICAL RESEARCH AUTHORITY, Salisbury, Wiltshire SP4 0JG (GB)
(72) Inventor: FARRAR, Graham Henry, Salisbury, Wiltshire SP4 0JG (GB); JONES, David Hugh, Salisbury, Wiltshire SP4 0JG (GB); CLEGG, James Christopher Stephen, Salisbury, Wiltshire SP4 0JG (GB)
(74) Representative: Dörries, Hans Ulrich
(86) International application number: PCT/GB1996/002770
(87) International publication number: WO 1997/017063

(56) References cited:
- EP-A1- 0 257 915
- EP-A2- 0 248 531
- EP-A2- 0 266 119
- EP-A2- 0 333 523
- WO-A-94/23738
- WO-A-95/20660
- WO-A-95/24929
- WO-A1-88/01213
- WO-A1-90/13361
- WO-A1-94/18954
- WO-A1-95/03356
- WO-A1-95/03357
- WO-A1-95/21931
- WO-A1-95/35097
- US- - 3 523 906
- US- - 4 262 090
- US- - 5 075 109
- US-A- 5 460 831
- ASAIO JOURNAL, vol. 40, no. 3, 1 July 1994, pages 584-589, XP000498246 RAJASUBRAMANIAN G ET AL: "FABRICATION OF RESORBABLE MICROPOROUS INTRAVASCULAR STENTS FOR GENE THERAPY APPLICATIONS"
- VACCINE, vol. 12, no. 1, 1 January 1994, pages 5-11, XP000578212 MORRIS W ET AL: "POTENTIAL OF POLYMER MICROENCAPSULATION TECHNOLOGY FOR VACCINE INNOVATION" cited in the application
- JOURNAL OF MICROENCAPSULATION, vol. 12, no. 1, 1 January 1995, pages 59-69, XP000486815 SAH H K ET AL: "BIODEGRADABLE MICROCAPSULES PREPARED BY A W/O/W TECHNIQUE: EFFECTS OF SHEAR FORCE TO MAKE A PRIMARY W/O EMULSION ON THEIR MORPHOLOGY AND PROTEIN RELEASE"
- PHARMACEUTICAL RESEARCH, vol. 8, 1991, page S151 XP002026578 NELLORE ET AL: "APPLICATION OF BIODEGRADABLE MICROSPHERES TO HEPATITIS B SURFACE ANTIGEN VACCINATION SYSTEM"
- BROWN T.A.: 'Gentechnologie für Einsteiger (2. Auflage)', pages 29 - 48
- COWSAR D.R. ET AL METHODS ENZYMOLOGY vol. 112, May 1985, pages 101 - 106
- ELDRIDGE J.H. ET AL INFECT. IMMUN. vol. 59, no. 9, September 1991, pages 2978 - 2986
- JONES D.H. ET AL DEV. BIOL. STAND. vol. 92, 1998, pages 149 - 155
- LEWIS K.J. ET AL J. CONTROLLED RELEASE vol. 37, 1995, pages 173 - 183
- TINSLEY-BOWN A.M. ET AL J. CONTROLLED RELEASE vol. 66, 2000, pages 229 - 241
- UCHIDA T. ET AL BIOL. PHARMA. BULL. vol. 17, no. 9, 1994, pages 1272 - 1276
- WHEELER ET AL INT. ARCH. ALLERGY APPL. IMMUN. vol. 83, 1987, pages 354 - 358
- CHANG ET AL J. BIOENGINEERING vol. 1, 1976, pages 25 - 32
- SPOONER ET AL GENE THERAPY vol. 2, 1995, pages 173 - 180
- WOLFF ET AL SCIENCE vol. 237, 1990, pages 1465 - 1468
- ELSNER H.I.; LINDBLAD E.B. DNA vol. 8, no. 10, 1989, pages 697 - 701
- ALLEN G.P.; RANDALL C.C. INFECTION IMMUNITY vol. 22, October 1978, pages 34 - 40

## Description

The present invention relates to methods of preparing microparticles containing DNA.

The bio-degradable polymer poly (DL-lactide-co-glycolide) (PLG) has been used for many years by the pharmaceutical industry to deliver drugs and biologicals in microparticulate form *in vivo.* The United States FDA has recently approved a PLG microsphere 30-day delivery system for leuprolide acetate (Lupran Depot (registered trade mark)) to be used in the treatment of prostate cancer. A useful review of the potential of polymer microencapsulation technology for vaccine use is found in Vaccine, 1994, volume 12, number 1, pages 5-11, by William Morris et al.

As an alternative to encapsulation, it is also known to deliver antigens in phospholipid vesicles called liposomes, as described for example by Eppstein, D.A et al in Crit. Rev. Ther. Drug Carrier Syst. 1988, 5(2), pages 99-139. It is reported that a number of antigens have been delivered intraperitoneally using liposomes, including cholera toxin, malaria sporozoite protein and tetanus toxoid, and that influenza antigen has been delivered intra-nasally.

It is also known that, in certain circumstances, injection of naked DNA into tissue can lead to expression of a gene product coded by that DNA. For example, in 1984, work at the United States NIH reported that intrahepatic injection of naked, cloned plasmid DNA for squirrel hepatitis produced both viral infection and the formation of anti-viral antibodies in the squirrels.

WO-A-95/05853 describes methods, compositions and devices for administration of naked polynucleotides which encode biologically active peptides. This published application describes, *inter alia,* the injection of naked DNA coding for an immunogenic antigen with the aim of raising antibodies in the recipient of the naked DNA.

Liposomal delivery of DNA is also known, and is described, for example, in EP-A-0475178.

An alternative method for obtaining expression of a desired gene product is described in EP-A-0161640, in which mouse cells expressing bovine growth hormone are encapsulated and implanted into a cow to increase milk production therein.

EP-A-0248531 describes encapsulating linear poly (I:C) in microcapsules and using these to induce production of interferon.

WO-A-94/23738 purports to describe a microparticle containing DNA in combination with a conjugate that facilitates and targets cellular uptake of the DNA. In working examples, bombardment of cells by microparticles containing Tungsten is described. These examples appear little different to conventional bombardment of cells with DNA-coated metal particles. Furthermore, sonication is proposed in microparticle manufacture, a step that is known to risk DNA damage but the presented data is inadequate and inappropriate to determine the integrity of the encapsulated DNA.

In the present invention, it is desired to deliver, *in vivo,* DNA encoding proteins with immunogenic, enzymatic or other useful biological activity, usually under the control of an active eukaryotic promoter. Objects of the invention include improvement on vaccination therapies known in the art and improvement upon prior art gene therapy methods.

Improvement of or alternatives to existing compositions and methods are desirable as these existing methods are known to contain a number of drawbacks.

WO-A-95/05853 describes administration of naked polynucleotides which code for desired gene products. However, the compositions and methods in this publication are suitable only for injection, requiring sterile procedures, being in itself an unpleasant and awkward route of administration.

WO-A-94/23738 purports to provide a process in which encapsulated DNA is released from the particles in the body of the recipient and then taken up by cells, although no accomplished *in vivo* examples are presented.

It is known that DNA is readily damaged so that it is no longer capable of inducing expression of a gene product. Surprisingly, the inventors have succeeded in devising a technique for encapsulation of DNA within polymer particles, such that the DNA retains sufficient integrity to induce expression of a gene product coded thereby.

The invention provides a method of encapsulating DNA in a polymer such that biological activity of the DNA is retained to a significant extent. In an embodiment of the second aspect, a method for encapsulating DNA within a polymer particle, said DNA being capable of inducing expression of a coding sequence within said DNA, comprises preparing a (water-in-oil)-in-water emulsion to form microparticles and separating subsequently produced DNA-containing microparticles by centrifugation. Resultant microparticles preferably have sizes in the range 0.01 µm to 10 µm, more preferably 1 µm to 10 µm.

The method of the invention is carried out under conditions that ensure at least a portion of the DNA is not damaged during manufacture of the particles and thereby retains its ability to induce expression of its gene coding sequence.

It is essential that DNA is incorporated into the microparticles, and DNA incorporation is increased by preparing a solution of DNA plus an alcohol, adding microparticle polymer and forming microparticles therefrom. The alcohol content of the solution suitably varies between 1% and 60% and preferably between 5% and 40%. In specific embodiments of the invention the alcohol content is around 15-35%, more particularly 20-30% for microparticles made from PLG, producing DNA incorporation of 25% and above, up to 50-60%. Ethanol is particularly suitable; methanol and propanol and other alcohols that do not denature DNA are also suitable, and the alcohol is preferably a straight chain or branched C₂-C₁₀ alcohol.

It is also preferred that the emulsification step or steps of the method be carried out under conditions of reduced shear stress, and this is optionally achieved by use of an emulsifying speed that is sufficient to obtain an emulsion and to form microparticles in the desired size range but not so high that all DNA is damaged by excessive shear. In an embodiment of the invention described below the emulsifying mixer speed is modified so that at least 25% DNA activity (assayed by transformation of competent bacteria or transfection of cultured cells) is retained in the resultant microparticles that contain DNA. Suitable speeds are below 8000 rpm, preferably below 6000 rpm, and in a specific embodiment described below the speed is about 3000 rpm.

The method may be performed at ambient temperature, which is convenient for laboratory and industrial purposes, and may also be performed at below ambient temperature improves the stability of the plasmid DNA during the encapsulation procedures. The temperature of the method may be reduced to below 20°C, below 10°C or even below 5°C. In an embodiment of the invention, the method is carried out at below ambient temperature using a reduced amount of microparticle precursor compared to the amount used at ambient temperature.

The parameters of the method are thus chosen to promote formation of microparticles of 10µm diameter or less and to promote incorporation of DNA into microparticles, and to avoid damage to the DNA such that the DNA can not be expressed in the recipient.

For any particular choice of polymer and DNA variations in the method may be necessary to obtain best results. The efficiency of a method can be assessed by transformation or transfection assays. In the transformation assay used by the inventors, DNA is recovered from microparticles by dissolution with organic solvent, quantitated and used to transform bacteria - ampicillin selection determines successful transformants. In the transfection assay, recovered DNA is used to transfect eukaryotic cells in culture, which culture is then assayed for presence of the antigen or gene therapy product. These assays have demonstrated that DNA recovered from microparticles produced by the method of the invention can retain 50-60% and up to 80% of the activity of the original DNA, indicating high efficiency of incorporation of functional DNA into microparticles.

In a further embodiment of the invention there is provided a method of making a pharmaceutical composition comprising preparing a DNA construct for expression of a coding sequence within the construct, and forming around the construct a polymer particle of size between 0.01µm and 10µm, wherein the construct remains capable of inducing expression of the coding sequence. In use, when the construct is separated from the particle, it induces expression of the coding sequence. The particle is preferably formed by emulsifying a solution of a polymer plus DNA plus alcohol.

The method of the invention is adapted to produce pharmaceutical compositions of the first aspect of the invention. The steps of the method are adapted so that, in a resultant composition which contains many DNA containing polymer particles, a useful proportion of particles contain active DNA, i.e. DNA that has not been damaged by the method such that its ability to induce expression of its coding sequence is lost. DNA activity is measured as a percentage of activity prior to the particle forming step.

An acceptable level of DNA biological activity is at least 10% and preferably at least 25%, though for particularly fragile DNA a lower percentage may be acceptable so long as, in use, a therapeutic effect is demonstrated by the composition.

In a specific embodiment of the invention, a composition is made by preparing a solution of a plasmid of double stranded, supercoiled DNA comprising a coding sequence and a eukaryotic promoter. Separately, a polymer solution is prepared. The two solutions are mixed together and emulsified at a speed between 1000 and 4000 rpm. A solution of a stabilizing agent is then added and the new mixture emulsified at a speed between 1000 and 4000 rpm. After centrifugation and resuspension of particles the DNA within retains 25% of its activity.

In a specific embodiment of the invention described in an example below, the particle material is PLG. The size of particles produced by the method of the invention are generally in the range of 0.01-10 µm, preferably 1-10 µm. Other suitable polymer formulations for DNA - containing particles according to the present invention include poly-lactic acid, poly - hydroxybutyrate, poly hydroxyvalerate, poly (hydroxybutrate/valerate), ethyl cellulose, dextran, polysaccharides, polyalkylcyanoacrylate, poly-methyl-methacrylate, poly(e- caprolactone) and mixtures of all of these components.

In use of a specific embodiment of the invention, described in an example below, a preparation of microparticles according to the invention comprises DNA coding for the protein luciferase. As will be appreciated by a person of skill in the art, a wide range of DNA sequences and constructs are suitable for use in this invention. In particular, the invention can be practised incorporating a wide range of plasmid vectors already well known and characterised in the art. Typically, a plasmid vector used in this invention will include a cDNA that codes for the desired gene product. The selection of additional components for the DNA sequence, such as promoters, reporter genes and transcription termination sequences can be made by a person of skill in the art according to common general knowledge concerning construction of known plasmid vectors.

It is known that uptake of microparticles of less than 10 µm in size occurs, *inter alia,* in the M cells of the intestine, and thus inclusion of DNA containing particles in this size range can be advantageous in promoting uptake at this intestinal location. Other modifications to the nature and character and components of the polymer can be made within the concept of the invention.

There now follows description of specific embodiments of the invention, accompanied by figures in which:-
Fig.1 is a schematic diagram of the components of a protein - expressing plasmid suitable for incorporation into a DNA-containing particle according to the invention;
Fig. 2 illustrates the results of example 3, namely induction of luciferase - specific serum antibodies by PLG - encapsulated plasmid DNA, and in which the left column indicates antibody titre after 3 weeks and the right column indicates antibody titre after 6 weeks (i.m. represents intra muscular, i.p. represents intra peritoneal, the bottom portion of each column represents IgG levels, the top portion represents IgM levels);
Fig. 3 shows dose-response data for injected and oral doses of encapsulated DNA, with the titre of IgG, IgM and IgA in each case.
Fig. 4 shows: A - agarose gel electrophoresis of plasmid DNA following homogenisation in a Silverson mixer at 2000 and 8000 rpm for 0 - 300 seconds; and B - agarose gel electrophoresis of DNA before and after encapsulation;
Fig. 5 shows stool anti-luciferase IgA response to DNA within PLG microparticles.
Fig. 6 shows the results of oral administration of PLG - encapsulated DNA expressing measles virus N protein; and
Fig. 7 shows the results of oral administration of PLG - encapsulated DNA expressing rotavirus VP6 gene: A - faecal rotavirus - specific IgA response, B - rotavirus shedding after challenge of orally immunized mice.

### Example 1

### Method for Encapsulation of Plasmid DNA in PLG microparticles Equipment:

1) Silverson Laboratory mixer with 3/4" probe fitted with emulsor screen.
2) High speed centrifuge.
3) Normal laboratory glassware, beakers, measuring cylinders, stirrers etc.

### Reagents:

1) Poly(lactide-co-glycolide) (PLG) solution - 500 mgs in 3 ml dichloromethane.
2) Plasmid DNA (12 mg/ml in water).
3) Polyvinyl alcohol (PVA) solution (8% w/v in water).
4) Absolute ethanol.
5) TEN buffer (10 mM tris pH 8.0 + 1 mM EDTA + 50 mM NaCl).

### Method:

1) Mix 200 µl plasmid DNA solution with 250 µl of TEN and add 150 µl ethanol with stirring. Mix well.
2) Add this mixture to 3 ml PLG solution and emulsify in the Silverson mixer at 3000 rpm for 2 min.
3) Add this emulsion to 100 ml PVA and emulsify at 3000 rpm for 2 min.
4) Add the double emulsion to 1 litre of water and stir vigorously for 1 min.
5) Distribute the suspension of microparticles in centrifuge containers and centrifuge at 10,000 x *gₐᵥ* for 30 mins.
6) Resuspend the microparticle pellet in 25ml of water and homogenise with a hand homogeniser with large clearance (0.5mm) to make a homogeneous suspension. Dilute with 200 ml of water and recentrifuge as above.
7) Repeat step 6 three times.
8) Resuspend the microparticle pellet in 25 ml of water as above, transfer to a vessel suitable for freeze drying, shell freeze in an isopropanol/dry ice mixture and lyophilise for 48 h.

In this method , steps 1-3 are carried out at ambient temperature. DNA was incorporated into the microparticles with an efficiency of about 25%.

### Example 2

### Plasmids for the Expression of Proteins after In Vivo Delivery

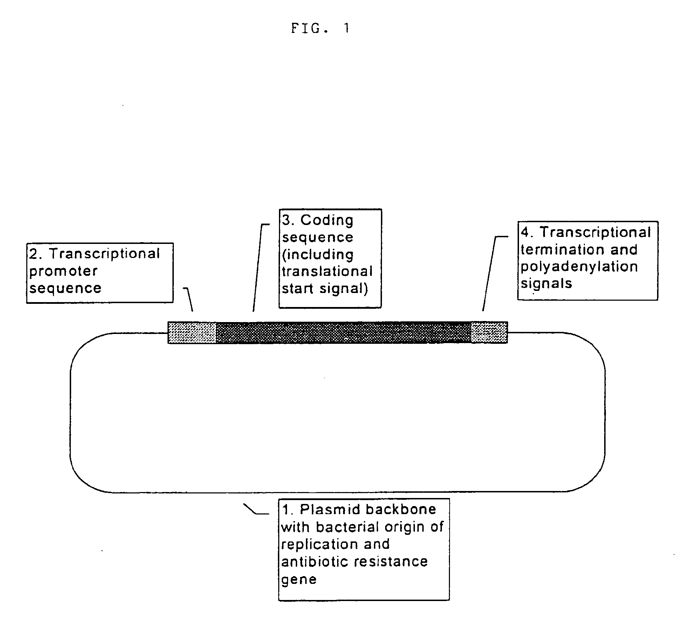

Suitable plasmids for use in microparticles according to the invention consist of the following components (see fig. 1):-
**1. Plasmid Backbone** The plasmid backbone has an origin of replication and an antibiotic resistance gene or other selectable marker to allow maintenance of the plasmid in its bacterial host. Backbones providing a high copy number will facilitate production of plasmid DNA. An example would be from the pUC plasmid vectors.
**2. Transcriptional Promoter** Sequence Expression of the desired protein will be driven by a, typically eukaryotic, transcriptional promoter initiating the synthesis of mRNA. Generally, strong promoters functioning in a wide variety of tissue types and animal species are to be used, e.g. the human cytomegalovirus immediate early (hCMV IE) promoter. However, particularly for gene therapy applications, a tissue- or cell type-specific promoter may be more appropriate.
**3. Coding Sequence** The coding sequence contains the DNA sequence encoding the protein of interest. It contains the translational start codon ATG in sequence context favourable for initiation of protein synthesis. The coding sequence ends with a translational termination codon. Proteins to be expressed include a) reporter enzymes (e.g. luciferase, β-galactosidase); b) components of pathogenic microorganisms capable of inducing protective immune responses (e.g. the NS1 protein of tick-borne encephalitis virus, the N, H or F proteins of measles virus, the gp120 protein of human immunodeficiency virus 1); c) enzymes or other proteins intended for the treatment of genetic disease (e.g. glucocerebrosidase for the treatment of Gaucher's disease).
**4. Transcription Termination Sequence** Improved expression levels are obtained under some circumstances when sequences causing termination of mRNA transcription are incorporated downstream of the coding sequence. These sequences frequently also contain signals causing the addition of a poly A tail to the mRNA transcript. Sequences which can be used in this role can be derived from the hCMV major immediate early protein gene or from SV40 viral DNA or elsewhere.

### Example 3

We have constructed a plasmid encoding the insect protein luciferase, under the transcriptional control of the human cytomegalovirus immediate early (hCMV IE) promoter, and demonstrated luciferase activity in cells transfected *in vitro.*

We encapsulated purified plasmid DNA in PLG microparticles around 2 µm in size with moderate (about 25%) efficiency using the protocol of Example 1. Agarose gel electrophoresis indicates that a proportion of the initially closed circular supercoiled DNA undergoes conversion to a more slowly migrating form, probably relaxed circles, as a result of shear stresses in the encapsulation process. The encapsulated DNA was released from the particles and shown to retain a significant fraction of its *in vivo* biological activity in assays of bacterial transformation by electroporation, and luciferase expression after transfection into cultured cells.

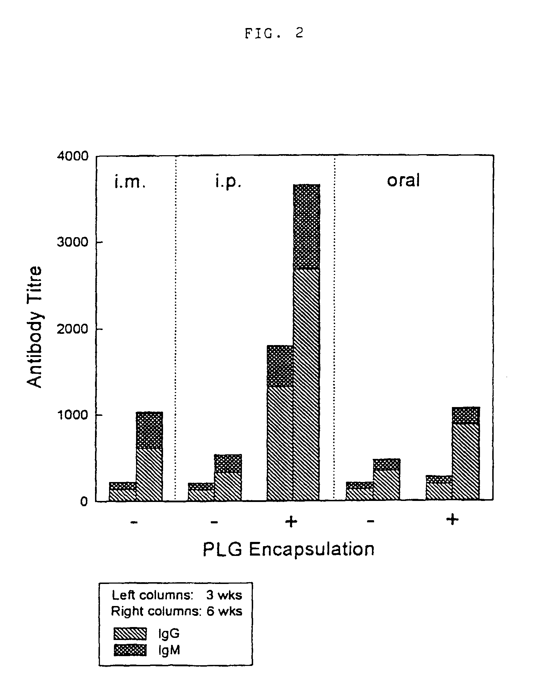

Microencapsulated DNA (50 µg) was administered to mice by intraperitoneal (i.p.) injection and orally. Control animals received unencapsulated DNA by the same routes, and as a positive control by standard intramuscular (i.m.) injection. Luciferase-specific serum antibodies were analyzed by ELISA three and six weeks after DNA administration. Results are presented in fig. 2.

As shown in fig. 2, modest specific IgG and IgM responses were seen after i.m. injection, as might be expected. Encapsulated DNA evoked strong IgG and IgM responses after i.p. injection, while unencapsulated DNA gave much weaker responses. Similarly, orally administered encapsulated DNA evoked a good IgG response which was not matched by unencapsulated DNA. The IgG and IgM antibody responses indicate that luciferase expression and presentation to the immune system occur after administration of plasmid DNA encapsulated in PLG microparticles with efficiencies exceeding that seen with the standard i.m. route, and exceeding that seen in comparative administration of unencapsulated DNA.

### Example 4

In further experiments, microencapsulated DNA, made by the method of Example 1, in a range of doses (1-50 µg DNA) was administered to groups of outbred mice by intra-peritoneal (i.p.) injection or orally. Luciferase-specific serum antibodies of IgG, IgM and IgA classes were analysed by ELISA at 3,6 and 9 weeks after DNA administration.

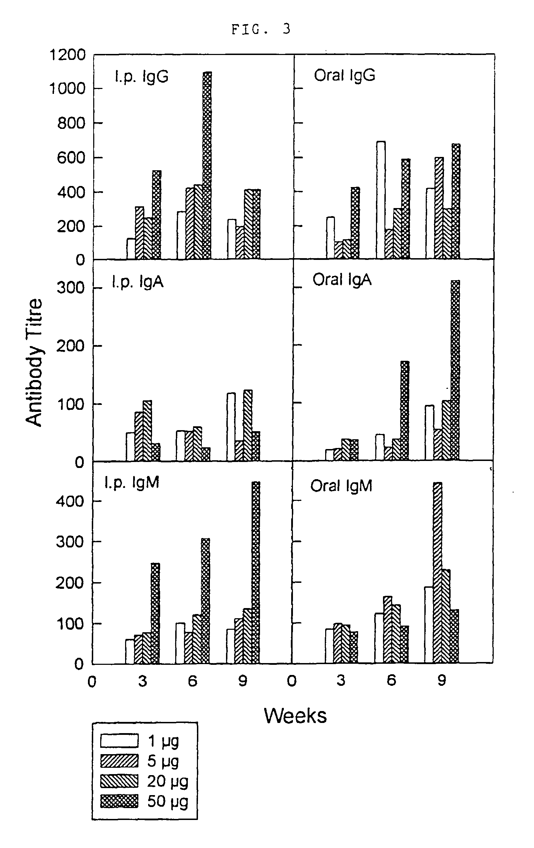

In Fig. 3, it can be seen that i.p. injection of PLG-encapsulated DNA evoked good IgG and IgM responses, and a modest IgA response. Orally administered encapsulated DNA evoked good responses in all three antibody classes. There is a trend for the antibody titres to increase with time after DNA administration, and the responses are also dose-related to a greater or lesser extent. It is apparent that quantities of DNA as low as 1 µg are able to evoke significant responses, especially at longer times after administration. These antibody responses again confirm that luciferase expression occurs after administration of plasmid DNA encapsulated in PLG microparticles, either by i.p. injection or orally. They also demonstrate that antigen is presented to the immune system by these means in such a fashion as to evoke IgG, IgM and IgA classes of antibody.

### Example 5

We examined the effect of high-speed homogenisation steps, used to generate the required water-oil-water emulsions which are intermediates in the encapsulation process, on the physical integrity and biological function of plasmid DNA.

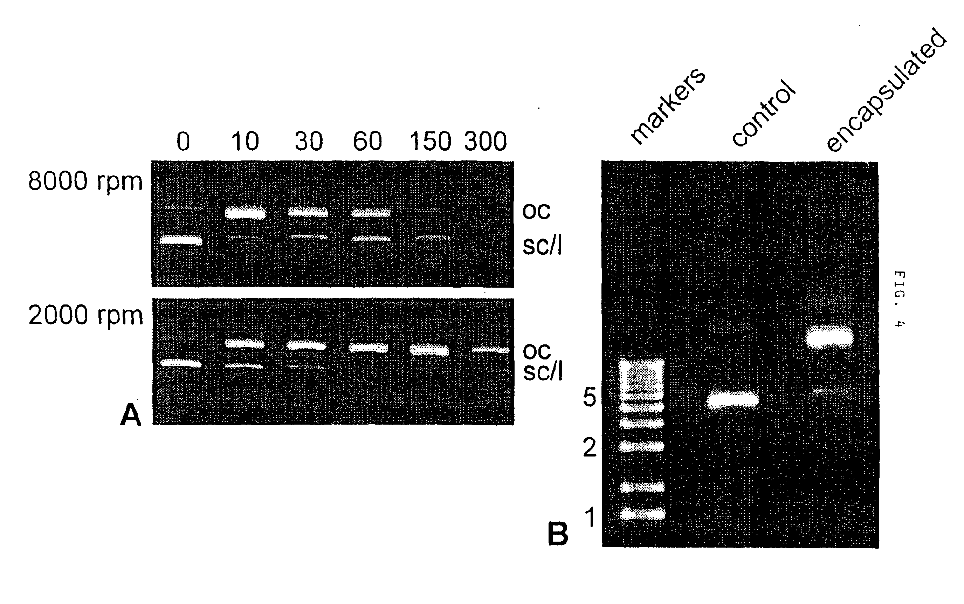

In initial experiments, supercoiled plasmid DNA was adjusted to concentrations and volumes similar to those to be used in microencapsulation experiments, and homogenised with a Silverson laboratory homogeniser. Samples were removed at intervals from 0 to 300 sec for analysis by agarose gel electrophoresis (Fig. 4A). Such an analytical procedure is capable of distinguishing between supercoiled (sc) DNA, open circular (oc) DNA, where a single strand has been nicked, and linear (I) DNA, where both strands have been cut at adjacent points (see, for example, Fig. 2C in Garner and Chrambach 1992. Resolution of circular, nicked and linear DNA, 4.4 kb in length, by electrophoresis in polyacrylamide solutions. Electrophoresis 13, 176-178). It is clear that exposure to such conditions for periods as short as 10 sec results in conversion from sc to oc form. At 8000 rpm, further conversion to the linear form and eventually more extensive degradation occur. However, at the reduced speed of 2000 rpm the oc form of DNA is relatively stable over the time period typically required for formation of the emulsion intermediates involved in PLG encapsulation. These studies thus show that plasmid DNA is vulnerable to shear-induced damage, and careful attention is required to the precise conditions to obtain encapsulation of minimally altered DNA.

From this basis, we have developed conditions for the encapsulation of purified plasmid DNA in PLG microparticles around 2 µm in size with moderate (about 25%) efficiency. Agarose gel electrophoresis (Fig. 4B) indicates that the initially closed circular supercoiled DNA undergoes conversion to the oc form, as a result of shear stresses in the encapsulation process. Biological activity of DNA released from microparticles has been assessed in assays of bacterial transformation by electroporation, and luciferase expression after transfection into cultured cells. DNA released from the particles retains a significant fraction (about 25%) of its *in vitro* activity in both these assays.

### Example 6

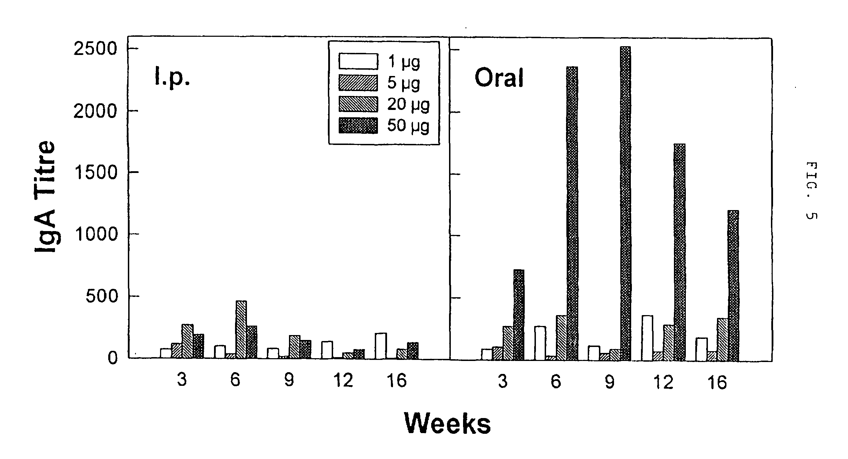

PLG-encapsulated DNA coding for luciferase, made by the method of Example 3, is also able to evoke a mucosal immune response to the expressed protein. Levels of IgG, IgM and IgA antibodies specific for luciferase were assessed by ELISA in stool samples from mice which received i.p. or oral doses of 1, 5, 20 or 50 µg of PLG-encapsulated DNA. No significant levels of IgG or IgM antibodies were found in stool samples from any group of mice. Rather limited IgA responses were seen in the i.p.-injected mice; however, oral administration resulted in significant levels of luciferase-specific IgA antibodies in the stool samples (Fig. 5). These reached extraordinarily high levels in those mice which received 50 µg PLG-encapsulated DNA. These results indicate that oral administration of a single dose of PLG-encapsulated plasmid DNA is capable of evoking a mucosal, as well as a systemic antibody response. This may be a useful attribute of a PLG-encapsulated DNA vaccine in applications where protection against infection at mucosal surfaces is desirable, as for measles or AIDS.

### Example 7

We have exploited a plasmid expressing the measles virus (MV) nucleocapsid protein (N) to extend our observations that the oral administration of encapsulated plasmid DNA expressing luciferase is capable of eliciting a systemic antibody response. The N-expressing construct is identical to that expressing luciferase (described in example 3), except for the replacement of the coding sequence with the Edmonston strain MV N coding sequence. The purified plasmid DNA was PLG-encapsulated (using the method as described in example 1).

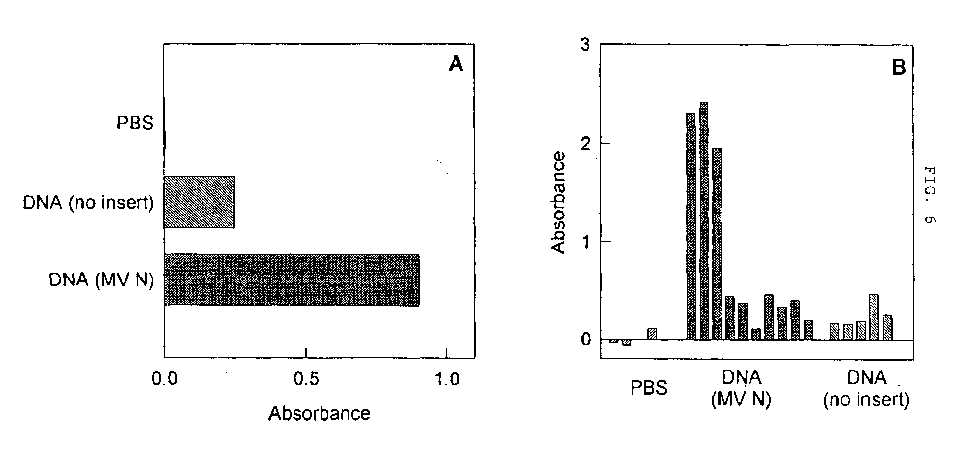

Inbred C3H mice were immunized with two doses suspended in 0.1 M Sodium bicarbonate administered by oral gavage, 13 days apart; each dose contained 50 µg DNA. Control groups of mice received PBS alone or PLG particles containing plasmid vector DNA containing no coding sequence. Mice were bled at intervals and serum levels of IgG specific for MV N determined by ELISA, using recombinant MV N expressed in insect cells as antigen. As shown in Fig. 6A, immunization with PLG-encapsulated DNA expressing MV N resulted in significant levels of N-specific antibody; results shown are mean absorbances in 1/100 diluted sera taken 53 days after the second DNA administration. There seems to be a considerable degree of variability in the response of individual mice to DNA immunization in these experiments (see Fig. 6B), but very high levels of antibody (reciprocal titres exceeding 10⁴, determined in follow-up experiments) are present in some animals. These results demonstrate that oral delivery of PLG-encapsulated DNA is an effective method for inducing an immune response against an important pathogen.

### Example 8

**A Further Method for Encapsulation of Plasmid DNA in PLG microparticles Equipment:**
1) Silverson Laboratory mixer with 3/4" probe fitted with emulsor screen.
2) High speed centrifuge.
3) Normal laboratory glassware, beakers, measuring cylinders, stirrers etc.

### Reagents:

1) Poly(lactide-co-glycolide) (PLG) solution - 500 mgs in 3 ml dichloromethane.
2) Plasmid DNA (>10 mg/ml in TE buffer).
3) Polyvinyl alcohol (PVA) solution (8% w/v in water).
4) Absolute ethanol.
5) TE buffer (10 mM tris pH 8.0 + 1 mM EDTA + 50 mM NaCI).

### Method:

1) Mix 450 µl plasmid DNA solution with 150 µl ethanol with stirring. Mix well.
2) Add this mixture to 3 ml PLG solution and emulsify in the Silverson mixer at 2000 rpm for 2½ min.
3) Add this emulsion to 100 ml PVA and emulsify at 2000 rpm for 2½ min.
4) Add the double emulsion to 1 litre of water and stir vigorously for 1 min.
5) Distribute the suspension of microparticles in centrifuge containers and centrifuge at 10,000 x *gₐᵥ* for 30 mins.
6) Resuspend the microparticle pellet in 25ml of water and homogenise with a hand homogeniser with large clearance (0.5mm) to make a homogeneous suspension. Dilute with 200 ml of water and recentrifuge as above.
7) Repeat steps 5 and 6 four times.
8) Resuspend the microparticle pellet in 25 ml of water as above, transfer to a vessel suitable for freeze drying, shell freeze in an isopropanol/dry ice mixture and lyophilise for 48 h.

In this method, steps 1-3 are carried out at ambient temperature. The efficiency was improved compared to example 1, up to 30-40% efficiency.

### Example 9

### A Further Method for Encapsulation of Plasmid DNA in PLG microparticles Equipment:

1) Silverson Laboratory mixer with 3/4" probe fitted with emulsor screen.
2) High speed centrifuge.
3) Normal laboratory glassware, beakers, measuring cylinders, stirrers etc.

### Reagents:

1) Poly(lactide-co-glycolide) (PLG) solution - 400 mgs in 3 ml dichloromethane.
2) Plasmid DNA (>10 mg/ml in TE buffer).
3) Polyvinyl alcohol (PVA) solution (8% w/v in water).
4) Absolute ethanol.
5) TE buffer (10 mM tris pH 8.0 + 1 mM EDTA.

### Method:

1) Mix 450 µl plasmid DNA solution with 150 µl ethanol with stirring. Mix well.
2) Add this mixture to 3 ml PLG solution and emulsify in the Silverson mixer at 2000 rpm for 2½ min.
3) Add this emulsion to 100 ml PVA and emulsify at 2000 rpm for 2½ min.
4) Add the double emulsion to 1 litre of water and stir vigorously for 1 min.
5) Distribute the suspension of microparticles in centrifuge containers and centrifuge at 10,000 x *gₐᵥ* for 30 mins.
6) Resuspend the microparticle pellet in 25ml of water and homogenise with a hand homogeniser with large clearance (0.5mm) to make a homogeneous suspension. Dilute with 200 ml of water and recentrifuge as above.
7) Repeat steps 5 and 6 four times.
8) Resuspend the microparticle pellet in 25 ml of water as above, transfer to a vessel suitable for freeze drying, shell freeze and lyophilise for 48 h.

In this method, steps 1-3 are carried out at 4°C. The efficiency of incorporation of DNA into microparticles was 50-60%.

### Example 10

Plasmid DNA (pCMVIA/VP6) expressing the VP6 gene of murine rotavirus (epizootic diarrhoea of infant mice (EDIM) virus) was constructed at the University of Massachusetts Medical Center. The gene encoding VP6 was inserted into a vector (pJW4303) downstream of sequences of the immediate early transcriptional promoter and intron A of human cytomegalovirus and a tPA-derived secretory signal sequence. The gene was followed by transcriptional termination and polyadenylation sequences derived from the bovine growth hormone gene. The purified plasmid DNA was PLG-encapsulated using the method described in example 1.

Inbred Balb/c mice were immunized by oral administration with a dose containing 50 micrograms VP6-expressing DNA encapsulated in PLG microparticles. A control group of mice received a similar dose of encapsulated vector DNA. Mice were examined for intestinal rotavirus-specific IgA at fortnightly intervals by ELISA, using EDIM virus as antigen. Nine weeks after immunisation, the animals were challenged with EDIM virus, and monitored for excretion of virus in stool using a monoclonal antibody-based ELISA.

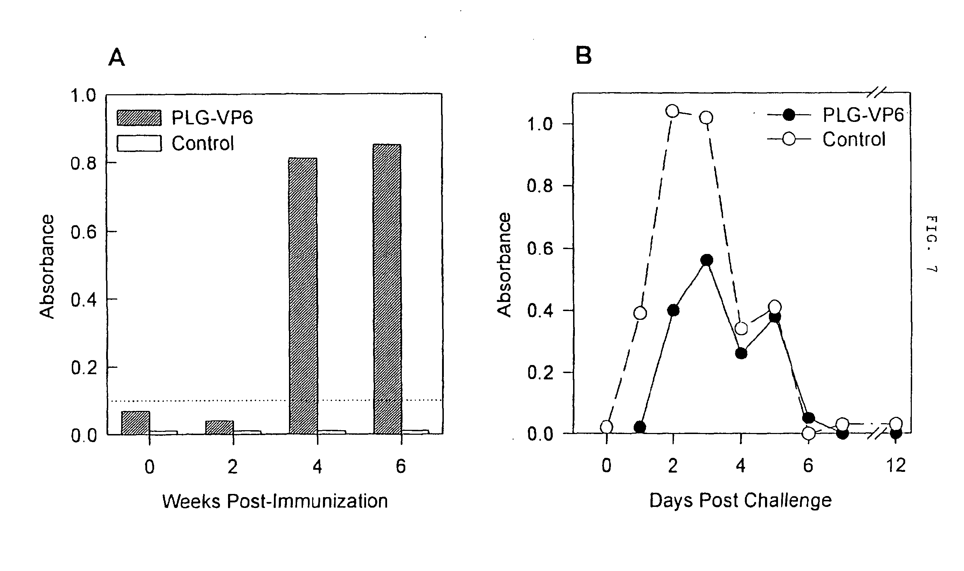

As shown in Fig. 7A, immunisation with PLG-encapsulated DNA expressing VP6 resulted in significant levels of rotavirus-specific intestinal IgA antibody when compared with the control animals. This is particularly noteworthy, since other routes of administration of VP6-expressing plasmid DNA do not elicit detectable levels of intestinal virus-specific IgA before virus challenge. After challenge with EDIM virus, there was a reduction in rotavirus shedding in mice which had received PLG - encapsulated VP6-expressing plasmid DNA, compared with the control group (Fig. 7B).

These results demonstrate that orally administered PLG-encapsulated plasmid DNA is capable of inducing a) a specific intestinal IgA response, and b) protection against virus challenge, as manifested in a reduction in virus shedding.

The compositions and methods of the invention have application in slow release systems for delivery of DNA vaccines; prolonged expression of immunogen potentially results in efficient single dose priming and boosting, with consequent efficient induction of long term memory responses. Another application is in a vehicle for the oral delivery of vaccines; simple and acceptable means for vaccine administration are likely to improve vaccine uptake rates; in addition, freeze-dried encapsulated plasmid DNA is likely to be very stable and insensitive to environmental conditions. A further application is in a slow release system for gene therapy; prolonged release of DNA and subsequent expression potentially reduces the need for repeated treatment.

## Claims

1. A method of encapsulating DNA in a polymer microparticle, wherein the DNA comprises a sequence coding for a polypeptide and is adapted to induce expression of the coding sequence, comprising the steps of preparing a mixture of the DNA and a water-in-oil-in-water emulsion suitable to form microparticles, forming microparticles of 10 µm or less in diameter that contain DNA and separating DNA-containing microparticles from the mixture by centrifugation, **characterised in that** the DNA retains its ability to induce expression of its coding sequence, and that at least 25% DNA activity, as assessed by an assay of transformation of competent bacteria or transfection of cultured cells, is retained in the resultant microparticles.

2. A method according to claim 1 comprising preparing a solution of DNA and alcohol.

3. A method according to any of Claims 1-2 comprising carrying out the method at 10°C or below.

## Patentansprüche

1. Verfahren zur Verkapselung von DNA in einem Polymer-Mikropartikel, wobei die DNA eine für ein Polypeptid codierende Sequenz umfasst und zur Induktion der Expression der codierenden Sequenz adaptiert ist, umfassend die Schritte: Herstellung eines Gemisches der DNA und einer Wasser-in-Öl-in-Wasser-Emulsion, die zur Bildung von Mikropartikeln geeignet ist, Herstellung von DNA-enthaltenden Mikropartikeln mit einem Durchmesser von 10 µm oder weniger und Trennung der DNA-enthaltenden Mikropartikel von dem Gemisch durch Zentrifugation, **dadurch gekennzeichnet, dass** die DNA ihre Eignung zur Induktion der Expression ihrer codierenden Sequenz beibehält, und dass in den resultierenden Mikropartikeln mindestens 25% DNA-Aktivität, gemäß eines Tests betreffend Transformation kompetenter Bakterien oder Transfektion kultivierter Zellen, erhalten bleibt.

2. Verfahren nach Anspruch 1, umfassend die Herstellung einer Lösung von DNA und Alkohol.

3. Verfahren nach einem der Ansprüche 1-2, umfassend die Durchführung des Verfahrens bei oder unter 10°C.

## Revendications

1. Méthode d'encapsulation d'ADN dans une microparticule polymère, dans laquelle l'ADN comprend une séquence codant pour un polypeptide et est adapté pour induire l'expression de la séquence codante, comprenant les étapes de: préparation d'un mélange de l'ADN et d'une émulsion eau dans l'huile dans l'eau approprié pour la formation des microparticules, formation des microparticules contenantes de l'ADN de 10 µm de diamètre ou moins et séparation des microparticules contenantes de l'ADN du mélange par centrifugation, **caractérisée en ce que** l'ADN conserve sa capacité d'induire l'expression de sa séquence codante, et **en ce qu'**au moins 25% activité de l'ADN, comme évaluée par un essai de transformation de bactéries compétentes ou transfection de cellules en culture, est retenu dans les microparticules ainsi obtenues.

2. Méthode selon la revendication 1, comprenant préparation d'une solution d'ADN et de l'alcool.

3. Méthode selon l'une quelconque des revendications 1-2, comprenant mise en oeuvre de la méthode à 10°C ou moins.
